# EUROPÄISCHE PATENTSCHRIFT

(11) **EP 1 427 524 B1**
(45) Veröffentlichungstag und Bekanntmachung des Hinweises auf die Patenterteilung: **07.03.2007**
(21) Anmeldenummer: 02767439.9
(22) Anmeldetag: 27.08.2002
(51) Int. Cl.: B01J 20/18, B01D 15/00, C07C 69/63, C07C 31/38, H01M 10/40

(54) **TRIFLUORETHANOLABTRENNUNG**
ISOLATION OF TRIFLUOROETHANOL
SEPARATION DE TRIFLUOROETHANOL

(30) Priorität: 04.09.2001 DE 10143170
(43) Veröffentlichungstag der Anmeldung: 16.06.2004
(73) Patentinhaber: Solvay Fluor GmbH, 30173 Hannover (DE)
(72) Erfinder: BÖSE, Olaf, 30167 Hannover (DE); PETERKORD, Katja, 30175 Hannover (DE)
(74) Vertreter: Jacques, Philippe
(86) Internationale Anmeldenummer: PCT/EP2002/009546
(87) Internationale Veröffentlichungsnummer: WO 2003/020419

(56) Entgegenhaltungen:
- EP-A- 0 696 077
- EP-A- 0 835 858
- US-A- 3 335 173
- US-A- 4 859 793
- US-A- 6 107 529
- DATABASE WPI Section Ch, Week 199250 Derwent Publications Ltd., London, GB; Class E16, AN 1992-411563 XP002221125 & JP 04 308537 A (ASAHI GLASS CO LTD), 30. Oktober 1992 (1992-10-30)

## Beschreibung

Die Erfindung bezieht sich auf ein Verfahren zur Abtrennung von Trifluorethanol aus organischen Flüssigkeiten.

Trifluorethanol kann als Lösungsmittel verwendet werden, es kann auch als Baustein in der chemischen Synthese, beispielsweise zur Herstellung von Estern, die ihrerseits dann als Lösemittel oder Synthesebaustein brauchbar sind, verwendet werden. Organische Flüssigkeiten können deshalb, beispielsweise herstellungsbedingt oder als Folge von Zersetzungsreaktionen, Trifluorethanol enthalten. Die Abtrennung des Trifluorethanols gestaltet sich in der Technik oft schwierig. Beispielsweise kann der Gehalt sehr niedrig sein, so daß bei einer Destillation Verluste an Ausbeute der organischen Flüssigkeit auftreten. Mit manchen organischen Flüssigkeiten, beispielsweise mit dem Trifluoressigsäuretrifluorethylester, bildet Trifluorethanol ein Azeotrop und kann deshalb destillativ überhaupt nicht abgetrennt werden. Aufgabe der vorliegenden Erfindung ist es, ein einfaches und wirksames Verfahren zur Abtrennung von Trifluorethanol (2,2,2-Trifluorethanol) aus organischen Flüssigkeiten anzugeben. Diese Aufgabe wird durch die folgende Erfindung gelöst.

Das erfindungsgemäße Verfahren zur Entfernung von Trifluorethanol aus organischen Flüssigkeiten, die mit Trifluorethanol verunreinigt sind, sieht vor, daß man die verunreinigten organischen Flüssigkeiten mit Molekularsieb einer Porengröße von 0,5 bis 1 nm kontaktiert.

Das erfindungsgemäße Verfahren kann im Prinzip für beliebige organische Flüssigkeiten verwendet werden, wobei der anzuwendende Druck keine Rolle spielt. Vorteilhaft ist es natürlich, das Verfahren bei Umgebungsdruck durchzuführen. Der Fachmann kann leicht feststellen, ob das erfindungsgemäße Verfahren, angewendet auf eine bestimmte organische Flüssigkeit, funktioniert. Er entnimmt einfach nach z. B. einer oder zwei Stunden eine Probe und überprüft diese gaschromatographisch daraufhin, ob der Gehalt an Trifluorethanol sich verringert hat.

Bevorzugt wendet man das erfindungsgemäße Verfahren auf Ether und besonders auf Ester des Trifluorethanols an, die mit Trifluorethanol verunreinigt sind. Bevorzugt entfernt man das Trifluorethanol aus mit Trifluorethanol verunreinigten Estern der Kohlensäure oder von Carbonsäuren. Ebenso reinigen lassen sich Phosphorsäureester oder Phosphonsäureester, die mit Trifluorethanol verestert sind, z. B. die in der EP-A 696 077 oder 0 806 804 beschriebenen Tris(trifluorethyl)phosphate oder die Phosphonsäureester und Phosphinsäureester, die in der US-A 6,210,840 beschrieben werden. Die Phosphon- und Phosphinsäuren, die dort beschrieben sind, weisen direkt an den Phosphor gebundene Alkyl-, Halogenalkyl-, Aryl-, Aralkyl- oder CH₂CO(O)OR-Gruppen auf. Hier ist R z. B. Alkyl oder halogeniertes Alkyl.

Bevorzugte Carbonsäureester, die erfindungsgemäß gereinigt werden, sind Ester der Essigsäure, Propionsäure, Butancarbonsäure, Pentancarbonsäure, Hexancarbonsäure und Heptancarbonsäure. Die genannten Ester können beispielsweise durch Halogenatome, vorzugsweise mindestens 1 Fluoratom, oder andere Substituenten wie C₁-C₄-Alkoxygruppen substituiert sein. Ganz besonders bevorzugt reinigt man Trifluorethylester der Kohlensäure, der Trifluoressigsäure, der Perfluorpropionsäure oder der Perfluorbutansäure.

Ganz besonders bevorzugt als Molekularsieb ist Molekularsieb 13X, welches eine Porengröße von 0,85 nm besitzt.

Die Abtrennung wird zweckmäßig bei Umgebungstemperatur durchgeführt, man kann aber auch bei niedrigerer Temperatur, beispielsweise bei Temperaturen bis zu 0 °C oder darunter, oder auch bei höheren Temperaturen, gewünschtenfalls bis hin zum Siedepunkt der organischen Flüssigkeit oder des Trifluorethanols, arbeiten.

Das erfindungsgemäße Verfahren weist folgende Vorteile auf:

Die wasserfreie Entfernung von Trifluorethanol in organischen Flüssigkeiten und Lösungsmitteln ist möglich, ohne das eine destillative Trennung nötig ist. Das Molekularsieb kann durch Dekantieren oder Filtrieren entfernt werden, wobei das Verfahren über einen weiten Konzentrationsbereich anwendbar ist. Die zu reinigende organische Flüssigkeit wird nicht kontaminiert. Da kein Wasserzusatz nötig ist, ist das Verfahren auch bei hydrolyseempfindlichen Substanzen anwendbar.

Es eignet sich besonders für die Anwendung von Lösungsmitteln, die in hoher Reinheit in der Elektrotechnik, beispielsweise als Elektrolytlösemittel für Lithiumionen-Batterien oder für Doppelschichtkondensatoren benötigt werden.

Die folgenden Beispiele sollen die Erfindung weiter erläutern, ohne sie in ihrem Umfang einzuschränken.

### Beispiele

### Beispiel 1 :

### Reinigung von Trifluoressigsäuretrifluorethylester

Aufgrund einer Bildung eines Azeotrops konnte Trifluorethanol nicht destillativ aus Trifluoressigsäuretrifluorethylester abgetrennt werden. Zur Entfernung des Trifluorethanols (0,479 Flächen-%, GC) aus Trifluoressigsäuretrifluorethylester (10 g; 196 g mol⁻¹; 0,051 mol) wurde jeweils eine Probe mit je 1 g Molekularsieb kontaktiert. Die entsprechenden Gehalte an Trifluorethanol nach der Behandlung können der Tabelle 1 entnommen werden.

**Tabelle 1: Gehalte an Trifluorethanol (GC, Flächen-%) in Trifluoressigsäuretrifluorethylester nach Behandlung mit verschiedenen Molekularsieben**

| Beispiel | verwendetes Molekularsieb | Kontaktzeit 1 h Gehalt TFE [Flächen-%] | Kontaktzeit 10 h Gehalt TFE [Flächen-%] |
|---|---|---|---|
| 1.1. | Molekularsieb 4A | 0,455 | 0,435 |
| 1.2. | Molekularsieb 5A | 0,377 | 0,100 |
| 1.3. | Molekularsieb 13X | 0,205 | 0,021 |

### Beispiel 2:

### Reinigung von Bis(trifluorethyl)carbonat

Bis(trifluorethyl)carbonat (10 g; 226 g mol⁻¹; 0,044 mol) mit einem Gehalt an Trifluorethanol von 2,47 % (GC, Flächen-%) wurde mit 1 g Molekularsieb 13X kontaktiert. Der Gehalt an Trifluorethanol betrug nach 2 h 0,82 % und nach 12 h 0,27 %.

Die Beispiele zeigen, daß Molekularsieb 13X besonders gut zur Abtrennung von 2,2,2-Trifluorethanol geeignet ist.

## Patentansprüche

1. Verfahren zur Entfernung von Trifluorethanol aus organischen Flüssigkeiten, die mit Trifluorethanol verunreinigt sind, **dadurch gekennzeichnet, daß** man die verunreinigten organischen Flüssigkeiten mit Molekularsieb einer Porengröße von 0,5 bis 1 nm kontaktiert.

2. Verfahren nach Anspruch 1, **dadurch gekennzeichnet, daß** man Ester des Trifluorethanols einsetzt, die mit Trifluorethanol verunreinigt sind.

3. Verfahren nach Anspruch 2, **dadurch gekennzeichnet, daß** man mit Trifluorethanol verunreinigte Ester der Kohlensäure, von Carbonsäuren, der Phosphorsäure, von Phosphon- oder Phosphinsäuren reinigt.

4. Verfahren nach Anspruch 3, **dadurch gekennzeichnet, daß** man mit Trifluorethanol verunreinigte Ester der Essigsäure, Propionsäure und Butancarbonsäure, die bevorzugt durch mindestens 1 Fluoratom substituiert sind, reinigt.

5. Verfahren nach Anspruch 1, **dadurch gekennzeichnet, daß** man mit Trifluorethanol verunreinigte Ester der Kohlensäure, der Trifluoressigsäure, der Pentafluorpropionsäure oder der Heptafluorbutansäure reinigt.

6. Verfahren nach Anspruch 1, **dadurch gekennzeichnet, daß** man Molekularsieb 13X, welches einer Porengröße von 0,85 nm aufweist, einsetzt.

## Claims

1. Method for the removal of trifluoroethanol from organic liquids which are contaminated with trifluoroethanol, **characterized in that** the contaminated organic liquids are contacted with a molecular sieve with a pore size of 0.5 to 1 nm.

2. Method according to claim 1 **characterized in that** esters of trifluoroethanol are used which are contaminated with trifluoroethanol.

3. Method according to claim 2 **characterized in that** esters of carbonic acid, of carboxylic acids, of phosphoric acid, of phosphonic or of phosphinic acid, which are contaminated with trifluoroethanol, are purified.

4. Method according to claim 3 **characterized in that** esters of acetic acid, propionic acid and butanecarboxylic acid, which preferably are substituted by at least 1 fluorine atom, which are contaminated with trifluoroethanol, are purified.

5. Method according to claim 1 **characterized in that** esters of carbonic acid, of trifluoroacetic acid, of pentafluoropropionic acid or of heptafluorobutanoic acid, which are contaminated with trifluoroethanol, are purified.

6. Method according to claim 1 **characterized in that** molecular sieve 13X is used which has a pore size of 0.85 nm.

## Revendications

1. Procédé d'élimination du trifluoroéthanol à partir de liquides organiques qui sont contaminés par du trifluoroéthanol, **caractérisé en ce que** l'on met en contact des liquides organiques contaminés avec un tamis moléculaire ayant une taille des pores de 0,5 à 1 nm.

2. Procédé selon la revendication 1, **caractérisé en ce que** l'on met en oeuvre un ester du trifluoroéthanol qui est contaminé par du trifluoréthanol.

3. Procédé selon la revendication 2, **caractérisé en ce que** l'on purifie un ester d'acide carbonique, d'acides carboxyliques, d'acide phosphorique, d'acide phosphonique ou phosphinique contaminé par du trifluoroéthanol.

4. Procédé selon la revendication 3, **caractérisé en ce que** l'on purifie un ester d'acide acétique, propionique ou butyrique, contaminé par du trifluoroéthanol, qui est substitué avec au moins un atome de fluor.

5. Procédé selon la revendication 1, **caractérisé en ce que** l'on purifie un ester d'acide carbonique, d'acide trifluoroacétique, d'acide pentafluoropropionique ou d'acide heptafluorobutyrique, contaminé par du trifluoroéthanol.

6. Procédé selon la revendication 1, **caractérisé en ce que** l'on met en oeuvre un tamis moléculaire 13X ayant une taille des pores de 0,85 nm.
